Europäisches Patentamt

European Patent Office

Office européen des brevets

(19)

(11) Publication number: **0 020 532**
**B1**

(12) **EUROPEAN PATENT SPECIFICATION**

(45) Date of publication of patent specification: **13.07.83**

(21) Application number: **79901407.1**

(22) Date of filing: **27.09.79**

(86) International application number:
**PCT/US79/00795**

(87) International publication number:
**WO 80/00660 17.04.80 Gazette 80/8**

(51) Int. Cl.³: **A 61 K 31/685,**
**A 61 K 31/13**

(54) **LECITHIN-TRIETHANOLAMINE SKIN PREPARATION.**

(30) Priority: **27.09.78 US 946175**

(43) Date of publication of application:
**07.01.81 Bulletin 81/1**

(45) Publication of the grant of the patent:
**13.07.83 Bulletin 83/28**

(84) Designated Contracting States:
**AT CH DE FR GB LU NL SE**

(56) References cited:
**DE - A - 2 740 346**
**FR - A - 1 439 040**
**GB - A - 505 983**
**US - A - 3 062 721**
**US - A - 3 957 971**

**N. Chemical Abstracts, Vol. 71, Issued 1969**
**Tegtmeier, Lecithin in Cosmetics, Siefen-Oele-**
**Fette-Wachse, 1969, 95 (7), 212 (Ger.) See**
**Abstract No. 42149b.**

(73) Proprietor: **Key Pharmaceuticals, Inc.**
**50 N.W. 176th Street**
**Miami, Florida 33169 (US)**

(72) Inventor: **FROST, Phillip**
**50 N.W. 176th Street**
**Miami, FL 33169 (US)**
Inventor: **Keith, Alec D.**
**50 N.W. 176th Street**
**Miami, FL 33169 (US)**

(74) Representative: **Laredo, Jack Joseph et al,**
**Elkington and Fife High Holborn House 52/54**
**High Holborn**
**London, WC1V 6SH (GB)**

Courier Press, Leamington Spa, England.

## Lecithin-triethanolamine skin preparation

This invention relates to skin preparations which are capable of a prolonged moisturising action and which provide a layer of protection at the surface of the skin and in immediately adjoining layers of the skin. The invention also relates to a drug release system for topical application to the skin of a patient requiring the drug contained in the system and to a burn preparation for topical application with improved results.

Background of the invention

U.S. Patent No. 3,957,971 is concerned with moisturising compositions for use as skin preparations. The solution there proposed relies on the use of so-called liposomes to remedy the problems of non-substantivity caused by high water solubility and loss of water migrating from the underlying skin when the conventional humectants are employed. The liposomes comprise a matrix of a lipid mixture having a plurality of cavities which contain a humectant in aqueous solution. Specifically, the patent proposes the use of a matrix of a ternary lipid mixture of lecithin, dicetyl phosphate and a sterol with internal cavities which contain a humectant dissolved in an aqueous medium. One example involves a mixture of soy bean lecithin, dicetyl phosphate and a sterol in predetermined molar ratios. Suitable humectants amongst a large number cited are glycerol, urea, sodium pyroglutamate, ornithine and various mixtures of amino-acids, acids and salts referred to as Spier-Pascher water solubles. The lecithin is employed to take advantage of its property of swelling in salt solutions, whilst the dicetyl phosphate imparts a negative charge to the lipid membranes so that the mutual repulsive action of opposing channel surfaces widens the channels and the sterol functions as an occlusive film when the liposomes are applied to skin or hair. Amongst the various examples there is a disclosure of a hand lotion composition which comprises a combination of an oil phase and a water phase. The oil phase comprises amerlate and glyceryl monostearate, whilst the water phase comprises triethanolamine and propylene glycol together with a predominant proportion of liposomes in aqueous medium. The liposomes referred to are prepared from a ternary mixture of 70 moles of soy bean lecithin, 20 moles of dicetyl phosphate and 10 moles of cholesterol followed by the addition of a sodium pyroglutamate solution and the removal thereafter by dialysis of any sodium pyroglutamate which is not bound in the liposomes. There is no teaching of the use of lecithin alone or in admixture with other phosphatides to achieve improved skin preparations, nor a teaching of the combination of lecithin with triethanolamine except in the context of the liposomes which are prepared from lecithin by reaction thereof with dicetyl phosphate and a sterol.

French Patent No. 1,439,040 is also concerned with cosmetic preparations in particular hair as opposed to skin preparations. The purpose of these compositions is particularly to supply the hair with lecithin, a well known material in the treatment of hair. Various examples of such lotions are given which comprises mixtures of lecithin with triethanolamine oleate or laurate. Although a broad reference is made to body treatment, there is no specific mention of use of these compositions as skin preparations, still less as drug release systems or as burn preparations for protecting burn victims from fluid loss. Although the proportions of triethanolamine oleate or laurate relative to the lecithin in the hair lotions vary in the examples given, there is no teaching corresponding to the particular discovery of the invention relating to specified proportions of lecithin and triethanolamine.

U.S. Patent No. 3,062,721 refers broadly to cosmetic base compositions, which comprise lecithin in admixture with various other components, such as borax, alfalfa, papain, aloe, sarsaparilla and menthol in aqueous $C_2$—$C_3$ alkanol solvent systems. The various ingredients are said to be essential and to display a synergistic action in combination with one another.

Werner Tegtmeier in an article published in Seifen-Öle-Fette-Wachse-95. Jg.-Nr. 7/1969, page 212, entitled Lecithin in der Kosmetik discusses lecithin as the most important of the phosphatides. Specifically, the author explains that the value of lecithin in cosmetics consists in a reduction of surface tension and the promotion of resorption, absorption and penetration. Thus, lecithin is stated to be an effective re-oiling agent for skin and hair and to have softening properties. Specifically, the author states that up to 4% of lecithin may be added to cosmetic preparations and mentions proportions of 1 to 2% in shampoos and as much as 10% in skin oils Combinations with cholesterol are also mentioned. On the other hand, the author states that in the case of dry skins it is advisable to use only from 0.5 to 0.65% because the skin dries out when too much lecithin is used. There is a teaching of the combination of lecithin with cholesterol derivatives and vaseline to increase water binding power, but there is no mention of any combinations with triethanolamine.

The Formularly Drogisten-Lexikon 1958, pages 327 and 348 teaches various proportions of triethanolamine in skin preparations varying from 1.2% up to 12%. Again, there is no teaching of any combination of lecithin with triethanolamine, still less of specific relative molar proportions.

Finally, German Patent Publication (As-Laid-Open) No. 27 40 346 is concerned broadly with various pharmaceutical compositions which are based on anthocyanidines and methods of preparation thereof. The inventors state as part of their discovery their findings regarding the healing powers of anthocyanidines which make them particularly suitable in pharmaceutical preparations of different kinds. A large number of additional components well known in the pharmaceutical field may form part of these compositions and amongst such lengthy recitals there is a teaching of the presence of lecithin in such pharmaceutical compositions. Furthermore, amongst the compositions described by way of example there are teachings of ointments for example which comprise in addition to the active anthocyanidine components, both lecithin and triethanolamine amongst other additives. There is no special teaching regarding the advantages or particular features of such combinations of lecithin with triethanolamine and there is no teaching whatsoever of the special proportions in which such components must be present to have their particular effectiveness in a drug release system. The main emphasis of the teaching as a whole is directed to the use of anthocyanidines in pharmaceutical compositions because of the particular properties of this drug which the inventors have discovered.

Summary of the invention

In a first aspect of the invention there is provided a skin preparation comprising an aqueous mixture of 1 to 5% by weight lecithin, said lecithin being optionally replaced by a lecithin containing mixture and at least an equimolar amount of triethanolamine which is 0.2 to 3% by weight of the total composition. A soy lecithin has been found to be practical for use as the lecithin of the present invention. A thickening agent such as carboxymethylcellulose may be incorporated into the skin preparation of the present invention, as may an emollient such as polyoxypropylene fatty ether of isopropylpalmitate.

In a preferred embodiment, the skin preparation of the present invention is an aqueous mixture which comprises 1 to 4% by weight lecithin; 0.2 to 3% by weight triethanolamine, provided that at least an equimolar amount of triethanolamine is provided based upon the amount of lecithin; 2 to 6% by weight emollient selected from the group consisting of a polyoxypropylene fatty ether, isopropylpalmitate and mixtures thereof; 0.5 to 2% by weight of a thickening agent; and 0.1 to 2.0% by weight citric acid.

The preparation of the present invention may be drug release system for topically delivering drugs to a patient in an uniform manner. The drug release system consists essentially of an aqueous mixture comprising from 1 to 5%, e.g. 3 to 5% preferably 4% by weight of lecithin, said lecithin being optionally replaced by a lecithin containing mixture, from 0.2 to 3% by weight triethanolamine, and a drug which is to be topically applied to the patient.

Hydrocortisone is an example of a drug which may be delivered to a patient through this aspect of the invention.

The present invention also provided preparations, for protecting a burn victim from fluid loss, when the skin preparation of the present invention is topically applied to the burn victim a film coating is produced on the skin, which permits oxygen exchange through skin, while at the same time retarding fluid loss from the skin tissue.

The present invention provides a skin preparation based upon the discovery that a mixture of lecithin with at least an equimolar amount of triethanolamine is particularly suited for keeping skin moist. Although the extract theory of the invention has not been conclusively established, the inventors believe that the lecithin-triethanolamine preparation provided via the present invention may not deeply penetrate the skin, or at least a significant portion stays at the outer area near or at the skin surface, the very area which is subject to dryness and flakiness. Due to the chemistry of lecithin, which has a positive and a negative charge site, the lecithin is believed to at least partially remain near the skin. In addition, each molecule of lecithin closely bonds 10 to 12 molecules of water, to the extent that the nature of the water molecule is sufficiently altered so that the water does not freeze. At least one mole triethanolamine should be present in the mixture for every mole lecithin; although the theory has not been established conclusively, one explanation is that lecithin and triethanolamine may form a complex with each other.

The total amount of lecithin which is advantageously incorporated into the preparation of the invention is from 1 to 4% by weight of the total composition. Higher ranges may be used, although, in practice, preparations which include such a high percentage of lecithin suffer from stickiness. Even a 4% lecithin composition tends towards stickiness; however, a 4% lecithin composition may be of particular value, offsetting the stickiness, where a drug delivery system is contemplated. For example, it has been found particularly advantageous for the topical administration of hydrocortisone to include 1% hydrocortisone and 4% lecithin in a preparation of the present invention, where the topical application of hydrocortisone is desired. It is also contemplated that a major amount of the lecithin may be replaced by other phosphatides and a minor amount of oils which occur in the lecithins obtained from commercial sources. For example, soy lecithin, containing only 40% lecithin, may be used as the lecithin of the present invention, this soy lecithin containing other phosphatides and soy oil.

The amount of triethanolamine depends first of all upon the amount of lecithin that is used, with at least one mole triethanolamine being required for each mole lecithin. Accordingly, when 1% by weight lecithin is used, then the corresponding weight of triethanolamine is 0.2%. Accordingly, the weight amount of triethanolamine will have a minimum of 0.2% triethanolamine while the maximum may exceed the mole amount of lecithin, a maximum amount of triethanolamine of 3% being contemplated. In a preferred embodiment of the invention in a preparation suitable for skin application to overcome problems of dryness, 0.4% triethanolamine has been found useful.

The lecithin may be any of the compounds or mixtures thereof which are commonly considered to be "lecithin". Soy lecithin has been found satisfactory for applicants' purposes. The lecithin used in testing the present invention is a commercial preparation sold by Sigma Chemical Company as soy lecithin. It contains 40% lecithin, other phosphatides and some soy oil. This commercial soy lecithin was used unmodified as obtained from Sigma Chemical Company.

In addition to lecithin and triethanolamine, there may also be included other ingredients depending upon the intended use of the skin preparation of the present invention. Emulsifiers may be added to the composition for ease of application. Polyoxypropylene fatty ethers (Arlamol (Trade Mark), Atlas Chemical) and isopropylpalmitate have been advantageously incorporated into skin preparations of the present invention. For reasons of providing preparations suitable for being squeezed from a tube or other suitable container, a thickening agent such as carboxymethylcellulose may be added to the skin preparation of the present invention. It is also advantageous to include from 0.1 to 2% citric acid as a means of providing pH control. Ethanol may be provided as a component of the skin composition, ethanol having bacteriostatic action and providing a cooling effect upon application to the skin. The amount of ethanol is not critical; in one preparation, 20% ethanol was used although higher or lower amounts would also be possible for this optional ingredient.

It is often considered desirable to add a fragrance to the composition and it is specifically contemplated that in a preferred embodiment a fragrance is included in the skin preparation. One fragrance which has been successfully incorporated into the skin preparation of the present invention is Balsam (Trade Mark) 16.596.

The skin preparation of the present invention may also be used to treat burn victims. It has been found that thin, deformable layers of approximately 1/8" (3 mm) 1/100" (0.25 mm) thickness will form upon the topical application of the skin preparation of the present invention to a subject, which provides a strong flexible film. The resultant film is both permeable to atmospheric gas exchange and able to reduce the evaporative loss of water from the tissue surfaces. Films that are thinner can also be formed [ca. 1/300" (0.08 mm)].

The skin preparation of the present invention is also useful for the treatment of ichtyosis.

A number of patients with a dry skin condition have been found to have been helped by application of the skin preparation of the present invention. Accordingly, the use of the skin preparation of the present invention is particularly useful for application to the skin of patients having a particularly dry or crusty skin.

Example 1

The following skin preparation was made:

|  | % by weight |
|---|---|
| soy lecithin (Sigma Chemical Co., containing 40% lecithin, other phosphatides and some soy oil) | 2.0 |
| triethanolamine | 0.4 |
| Arlamol (Trade Mark) E (polyoxypropylene fatty ether of ICI America) | 2.0 |
| Isopropylpalmitate | 2.0 |
| Carboxymethylcellulose (Hercules (Trade Mark) 7H3SF) | 1.0 |
| Citric acid | 0.13 |
| Ethanol | 20 |
| Fragrance (Balsam (Trade Mark) 16.596) | 0.3 |
| Water | remainder |
|  | ——— |
|  | 100% |

The above skin preparation has been tested on the skin and found to provide a good moisturizing effect. The moisture barrier provided by the molecular complex (each mole of lecithin capable of complexing tightly with 10 to 12 molecules of water) and the lecithin staying near the surface due to the positively and negatively charged portions of the molecule, the skin preparation of the present invention provides relatively long protection for the skin against drying.

Further tests with a large proportion of triethanolamine to lecithin have shown that such compositions are also effective in accordance with the present invention.

Example 2

A drug delivery system may advantageously be based upon the lecithin-triethanolamine skin preparation of the present invention. An advantage of the lecithin-triethanolamine drug delivery system of the present invention is found for those drugs to be topically applied where it is desired that the drug be delivered in an uniform manner to the patient in an area near the skin surface. Due to the characteristics of the lecithin-triethanolamine skin preparation of the present invention, this objective is facilitated. The skin preparation is designed to disperse certain types of drugs such as steroids. Many steroids are non-covalently complexed by phospholipids. This complexing action results in clustering the steroid material in a stable form where one steroid molecule per one or more phospholipid molecules forms the stable complex. In this manner, the skin preparation should be more effective in dispersing an uniform application of steroids to the skin as compared with conventional topical applications. Also, it is expected that the release of steroids from this complex may require more time than is ordinarily involved in the release of steroids from ordinary solvents. Although the mechanism for the drug delivery system of the present invention has not been conclusively established, one explanation is that the steroid lecithin complex is expected to be retained in the topical zones of skin for a longer period of time than when ordinary solvents are used which do not complex the steroid material. The amount of lecithin in the skin preparation of this second aspect of the invention is up to 5%, e.g., 3% to 5% by weight, and preferably 4% by weight.

The following composition, which may also include optional ingredients mentioned previously, has the following percentage by weight:

| | |
|---|---|
| Lecithin | 4% |
| Triethanolamine | 0.8% |
| Hydrocortisone | 1% |

The above composition is advantageously used for the topical application not only of hydrocortisone but also for other drugs which may be topically applied, such as prednisone.

When used as a drug delivery system, it may be beneficial to include an antioxidant such as butylated hydroxytoluene (BHT) in small percentages (0.02—0.2%) to help stabilize the drug.

**Claims for the Contracting States: CH, DE, FR, GB, LU, NL SE**

1. A skin preparation comprising an aqueous mixture of from 1 to 5% by weight of lecithin based on the total composition, said lecithin being optionally replaced by a lecithin-containing phosphatide mixture and at least an equimolar amount of triethanolamine based upon the amount of lecithin present which is from 0.2 to 3% by weight of the total composition.

2. A skin preparation according to claim 1, characterised in that said lecithin is replaced by a lecithin-containing phosphatide mixture consisting essentially of a soy lecithin mixture containing 40% lecithin, other phosphatides and soy oil.

3. A skin preparation according to claim 1 or claim 2, characterised in that said preparation additionally comprises a thickening agent.

4. A skin preparation according to claim 3, characterised in that said thickening agent consists of carboxymethylcellulose.

5. A skin preparation according to any one of claims 1 to 4, inclusive, characterised in that said preparation additionally includes an emollient selected from the group consisting of polyoxyethylene fatty ethers and isopropylpalmitate.

6. A skin preparation according to claim 1 for the treatment of dry skin which is an aqueous mixture comprising from 1 to 4% by weight of lecithin, said lecithin being optionally replaced by a lecithin-containing mixture; from 0.2 to 3% by weight of triethanolamine, from 2 to 6% by weight of an emollient selected from the group consisting of polyoxypropylene fatty ethers, isopropylpalmitate and mixture thereof; from 0.5 to 2% by weight of a thickening agent; and from 0.1 to 2.0% by weight of citric acid.

7. A skin preparation according to claim 6, characterised in that said lecithin is replaced by a lecithin containing phosphatide mixture consisting of a soy lecithin mixture containing 40% of lecithin, other phosphatides and soy oil.

8. A skin preparation according to claim 6 or 7, characterised in that said thickening agent is carboxymethylcellulose.

9. A skin preparation according to any of claims 6 to 8, inclusive, characterised in that said preparation additionally include from 15 to 25% by weight of ethanol.

10. A skin preparation according to any of claims 6 to 9, inclusive, which additionally includes a fragrance.

11. A drug release system according to claim 1 for the topical application of a drug to the skin of a patient, said drug release system consisting essentially of an aqueous mixture containing from 3% to 5% by weight of lecithin, said lecithin being optionally replaced by a lecithin containing mixture; from 0.2 to 3% by weight of triethanolamine and a drug which is to be topically applied to the patient.

12. A drug release system according to claim 11, characterised in that the said lecithin or said lecithin-containing phosphatide mixture is present in said aqueous mixture in a proportion of 4% by weight based on said mixture.

13. A drug release system according to claim 11 or claim 12, characterised in that a lecithin-containing phosphatide mixture is employed as a lecithin component of said drug release system, said mixture consisting of a soy lecithin mixture contianing 40% lecithin, other phosphatides and soy oil.

14. A drug release system according to any one of claims 11 to 13, characterised in that said drug is contained in said aqueous mixture in a proportion of up to 1% by weight.

15. A drug release system according to any one of claims 11 to 14, characterised in that said triethanolamine is present in a proportion of 0.4% by weight.

16. A drug release system according to any one of claims 11 to 15, characterised in that said drug is hydrocortisone.

17. A drug release system according to any one of claims 11 to 16, characterised in that said drug is a labile drug and the aqueous mixture contains butylated hydroxytoluene (BHT) in a proportion of from 0.02% to 0.2% as an antioxidant present to protect said labile drug.

18. A burn preparation according to claim 1 for use in protecting a burn victim from fluid loss by a topical application of said preparation to the skin of said burn victim, to form a film thereon, said preparation consisting essentially of an aqueous mixture containing from 1% to 5% by weight of lecithin said lecithin being optionally replaced by a lecithin containing mixture from 0.2% to 3% by weight of tri-ethanolamine.

**Claims for the Contracting State: AT**

1. A method of preparing a skin preparation characterised in that from 1% to 5% by weight of a lecithin component selected from the group consisting of lecithin and lecithin containing phosphatide mixtures, in which at least 40% of said phosphatide mixture consists of lecithin, at least an equimolar amount of triethanolamine relative to said lecithin, corresponding to 0.2 to 3% by weight of said triethanolamine, and water are admixed together to constitute an aqueous mixture in which said lecithin component and said triethanolamine are present in the above stated percentages, based on the total composition or said aqueous mixture.

2. A method according to claim 1, characterised in that a soy lecithin mixture containing 40% of lecithin, other phosphatides and soy oil is selected as said lecithin component for admixture with said triethanolamine and said water to form said aqueous mixture.

3. A method according to claim 1 which comprises admixing together from 1 to 4% by weight of lecithin, said lecithin being optionally replaced by a lecithin containing mixture; from 0.2 to 3% by weight of triethanolamine, provided that at least an equimolar amount of triethanolamine is used based upon the amount of lecithin used; from 2 to 6% by weight of an emollient selected from the group consisting of polyoxypropylene fatty ethers, isopropylpalmitate and mixtures thereof; from 0.5 to 2% by weight of a thickening agent; from 0.1 to 2.0% by weight of citric acid and water to make up the remainder of the aqueous composition in which the latter components are used in the stated percentages by weight based on the total composition including the make-up water.

4. A method according to claim 3, characterised in that the lecithin component used is a soy lecithin mixture containing 40% of lecithin, other phosphatides and soy oil.

5. A drug release system prepared according to claim 1 for the topical application of a drug to the skin of a patient, said drug release system consisting essentially of an aqueous mixture containing from 3% to 4% by weight, of lecithin said lecithin being optionally replaced by a lecithin containing mixture; from 0.2 to 3% by weight of triethanolamine and a drug which is to be topically applied to the patient.

6. A drug release system according to claim 5, characterised in that the said lecithin or said lecithin containing phosphatide mixture is present in said aqueous mixture in a proportion of 4% by weight based on said mixture.

7. A drug release system according to claim 5 or 6, characterised in that a lecithin-containing phosphatide mixture is employed as a lecithin component of said drug release system, said mixture consisting of a soy lecithin mixture containing 40% lecithin, other phosphatides and soy oil.

8. A drug release system according to any one of claims 5 to 7, characterised in that said drug is contained in said aqueous mixture in a proportion of up to 1% by weight.

9. A drug release system according to any one of claims 5 to 8, characterised in that said triethanolamine is present in a proportion of 0.4% by weight.

10. A drug release system according to any one of claims 5 to 9, characterised in that said drug is hydrocortisone.

11. A drug release system according to any one of claims 5 to 10, characterised in that said drug is labile drug and the aqueous mixture contain butylated hydroxytoluene (BHT) in a proportion of from 0.02% to 0.2% as an antioxidant present to protect said labile drug.

12. A burn preparation prepared according to claim 1 for use in protecting a burn victim from fluid loss by a topical application of said preparation to the skin of said burn victim to form a film thereon, said preparation consisting essentially of an aqueous mixture containing from 1% to 5% by weight of lecithin, said lecithin being optionally replaced by a lecithin containing mixture, from 0.2% to 3% by weight of triethanolamine.

**Revendications pour les Etats Contractants: CH, DE, FR, GB, LU, NL, SE**

1. Une préparation pour la peau comprenant un mélange aqueux de 1 à 5% en poids de lécithine, par rapport à la composition totale, ladite lécithine étant facultativement remplacée par un mélange de phosphatides contenant de la lécithine, et au moins une quantité équi-molaire de triéthanolamine, calculée par rapport à la quantité de lécithine présente, qui correspond à 0,2 à 3% en poids par rapport à la composition totale.

2. Une préparation pour la peau selon la revendication 1, caractérisée par le fait que ladite lécithine est remplacée par un mélange de phosphatides contenant de la lécithine consistant essentiellement en un mélange de lécithine de soja contenant 40% de lécithine, d'autres phosphatides et de l'huile de soja.

3. Une préparation pour la peau selon la revendication 1 ou la revendication 2, caractérisée en ce que ladite préparation comprend en outre un agent épaississant.

4. Une préparation pour la peau selon la revendication 3, caractérisée en ce que ledit agent épaississant consiste en carboxyméthyl-cellulose.

5. Une préparation pour la peau selon l'une quelconque des revendications 1 à 4, inclusivement, caractérisée en ce que ladite préparation comprend en outre un émollient choisi dans le groupe constitué par les éthers gras polyoxyéthylénés et la palmitate d'isopropyle.

6. Une préparation pour la peau selon la revendication 1 pour le traitement de peau sèche qui est un mélange aqueux comprenant de 1 à 4% en poids de lécithine, ladite lécithine étant facultativement remplacée par un mélange contenant de la lécithine; de 0,2 à 3% de triéthanolamine, de 2 à 6% en poids d'un émollient choisi dans le groupe constitué par les éthers gras polyoxypropylénés, le palmitate d'isopropyle et leurs mélanges; de 0,5 à 2% en poids d'un agent épaississant; et de 0,1 à 2,0% en poids d'acide citrique.

7. Une préparation pour le peau selon la revendication 6, caractérisée en ce que ladite lécithine est remplacée par un mélange de phosphatides contenant de la lécithine consistant en un mélange de lécithine de soja contenant 40% de lécithine, d'autres phosphatides et de l'huile de soja.

8. Une préparation pour la peau selon la revendication 6 ou 7, caractérisée en ce que ledit agent épaississant est la carboxyméthyl-cellulose.

9. Une préparation pour la peau selon l'une quelconque des revendications 6 à 8, inclusivement, caractérisée en ce que ladite préparation contient en outre de 15 à 25% en poids d'éthanol.

10. Une préparation pour la peau selon l'une quelconque des revendications 6 à 9, inclusivement, qui contient en outre un parfum.

11. Un système de libération de drogue selon la revendication 1 pour l'application topique d'une drogue sur la peau d'un patient, ledit système de libération de drogue consistant essentiellement en un mélange aqueux contenant de 3% à 5% en poids de lécithine, ladite lécithine étant facultativement remplacée par un mélange contenant de la lécithine; de 0,2 à 3% en poids de triéthanolamine et une drogue qui est destinée à être appliquée par voie topique au patient.

12. Un système de libération de drogue selon la revendication 11, caractérisé en ce que ladite lécithine ou ledit mélange de phosphatides contenant de la lécithine est présent dans ledit mélange aqueux dans une proportion de 4% en poids par rapport audit mélange.

13. Un système de libération de drogue selon la revendication 11 ou la revendication 12, caractérisé en ce qu'un mélange de phosphatides contenant de la lécithine est employé comme constituant de lécithine dudit système de libération de drogue, ledit mélange consistant en un mélange de lécithine de soja contenant 40% de lécithine, d'autres phosphatides et de l'huile de soja.

14. Un système de libération de drogue selon l'une quelconque des revendications 11 à 13, caractérisé en ce que ladite drogue est contenue dans ledit mélange aqueux dans une proportion allant jusqu'à 1% en poids.

15. Un système de libération de drogue selon l'une quelconque des revendications 11 à 14, caractérisé en ce que ladite triéthanolamine est présente dans une proportion de 0,4% en poids.

16. Un système de libération de drogue selon l'une quelconque des revendications 11 à 15, caractérisé en ce que ladite drogue est l'hydrocortisone.

17. Un système de libération de drogue selon l'une quelconque des revendications 11 à 16, caractérisé en ce que ladite drogue est une drogue labile et que le mélange aqueux contient de l'hydroxytoluène butylé (BHT) dans une proportion allant de 0,02% à 0,2% comme antioxydant présent pour protéger ladite drogue labile.

18. Une préparation pour brûlés, préparée selon la revendication 1, en vue d'être utilisée dans la protection d'une victime brûlée contre les pertes de fluide par application topique de ladite préparation sur la peau de ladite victime, pour former un film sur celle-ci, ladite préparation consistant essentiellement en un mélange aqueux contenant de 1% à 5% en poids de lécithine, ladite lécithine étant facultativement remplacée par un mélange contenant de la lécithine, de 0,2 à 3% en poids de triéthanol-amine.

**Revendications pour l'Etat Contractant: AT**

1. Une méthode d'obtention d'une préparation pour la peau, caractérisée par le fait que l'on mélange de 1 à 5% en poids d'un constituant de lécithine choisi dans le groupe constitué par la lécithine et les mélanges de phosphatides contenant de la lécithine, dans lesquels au moins 40% dudit mélange de phosphatides consiste en lécithine, au moins une quantité équimoléculaire de triéthanolamine par rapport à ladite lécithine, correspondant à 0,2 à 3% en poids de triéthanolamine, et de l'eau, pour constituer un mélange aqueux dans lequel ledit constituant de lécithine et ladite triéthanolamine sont présents dans les pourcentages indiqués ci-dessus, calculés par rapport à la composition totale ou audit mélange aqueux.

2. Méthode selon la revendication 1, caractérisée par le fait qu'un mélange de lécithine de soja contenant 40% de lécithine, d'autres phosphatides et de l'huile de soja est choisi comme constituant de lécithine pour être mélangé avec ladite triéthanolamine et ladite eau pour former ledit mélange aqueux.

3. Méthode selon la revendication 1, qui comprend les étapes constituant à mélanger ensemble de 1 à 4% en poids de lécithine, ladite lécithine étant facultativement remplacée par un mélange contenant de la lécithine; de 0,2 à 3% en poids de triéthanolamine, étant entendu qu'au moins une quantité équimoléculaire de triéthanolamine est utilisée, calculée par rapport à la quantité de lécithine utilisée; de 2 à 6% en poids d'un émollient choisi dans le groupe constitué par les éthers gras polyoxypropylénés, la palmitate d'isopropyle et leurs mélanges; de 0,5 à 2% en poids d'un agent épaississant; de 0,1 à 2,0% en poids d'acide citrique, le reste de la composition aqueuse étant constitué par de l'eau, les derniers constituants étant utilisés dans les pourcentages en poids indiqués, calculés par rapport à la composition totale y compris l'eau ajoutée.

4. Méthode selon la revendication 3, caractérisée par le fait que le constituant de lécithine utilisé est un mélange de lécithine de soja contenant 40% de lécithine, d'autres phosphatides et de l'huile de soja.

5. Un système de libération de drogue préparé selon la revendication 1, pour l'application topique d'une drogue sur la peau d'un patient, ledit système de libération de drogue consistant essentiellement en un mélange aqueux contenant de 3% à 4% en poids de lécithine, ladite lécithine étant facultativement remplacée par un mélange contenant de la lécithine; de 0,2 à 3% en poids de triéthanolamine et une drogue qui doit être appliquée par voie topique au patient.

6. Un système de libération de drogue selon la revendication 5, caractérisé en ce que ladite lécithine ou ledit mélange de phosphatides contenant de la lécithine est présent dans ledit mélange aqueux dans une proportion de 4% en poids par rapport audit mélange.

7. Un système de libération de drogue selon la revendication 5 ou 6, caractérisé en ce qu'un mélange de phosphatides contenant de la lécithine est employé comme constituant de lécithine dudit système de libération de drogue, ledit mélange consistant en un mélange de lécithine de soja contenant 40% de lécithine, d'autres phosphatides et de l'huile de soja.

8. Un système de libération de drogue selon l'une quelconque des revendications 5 à 7, caractérisé en ce que ladite drogue est contenue dans ledit mélange aqueux dans une proportion allant jusqu'à 1% en poids.

9. Un système de libération de drogue selon l'une quelconque des revendications 5 à 8, caractérisé en ce que ladite triéthanolamine est présente dans une proportion de 0,4% en poids.

10. Un système de libération de drogue selon l'une quelconque des revendications 5 à 9, caractérisé en ce que ladite drogue est l'hydrocortisone.

11. Un système de libération de drogue selon l'une quelconque des revendications 5 à 10, caractérisé en ce que ladite drogue est une drogue labile et que le mélange aqueux contient de l'hydroxytoluène butylé (BHT) dans une proportion allant de 0,02% à 0,2% comme antioxydant présent pour protéger ladite drogue labile.

12. Une préparation pour brûlés préparée selon la revendication 1, en vue d'être utilisée dans la protection d'une victime brûlée contre les pertes de fluide par application topique de ladite préparation sur la peau de ladite victime pour former un film sur celle-ci, ladite préparation consistant essentiellement en un mélange aqueux contenant de 1 à 5% en poids de lécithine, ladite lécithine étant facultativement remplacée par un mélange contenant de la lécithine, de 0,2% à 3% en poids de triéthanolamine.

**Patentansprüche für die Vertragsstaaten: CH, DE, FR, GB, LU, NL, SE**

1. Haut-Zubereitung (-Zusammensetzung) enthaltend eine wäßrige Mischung aus 1 bis 5 Gew.-% Lecithin bezogen auf die Gesamt-Zubereitung, wobei das Lecithin gewünschtenfalls durch eine lecithinhaltige Phosphatid-Mischung ersetzt ist, und mindestens einer äquimolaren Menge Triäthanolamin bezogen auf die vorhandene Menge Lecithin, welche 0,2 bis 3 Gew.-% der Gesamt-Zubereitung ausmacht.

2. Haut-Zubereitung nach Anspruch 1, dadurch gekennzeichnet, daß das Lecithin durch eine lecithinhaltige Phosphatid-Mischung ersetzt ist, welche im wesentlichen aus einer Sojalecithin-Mischung besteht, die 40% Lecithin, andere Phosphatide und Sojaöl enthält.

3. Haut-Zubereitung nach Anspruch 1 oder 2, dadurch gekennzeichnet, daß die Zubereitung zusätzlich ein Verdickungsmittel enthält.

4. Haut-Zubereitung nach Anspruch 3, dadurch gekennzeichnet, daß das Verdickungsmittel aus Carboxymethylcellulose besteht.

5. Haut-Zubereitung nach einem der Ansprüche 1 bis 4, dadurch gekennzeichnet, daß die Zubereitung zusätzlich ein Erweichungsmittel umfaßt, das aus der aus Polyoxyäthylen-Fett-Äther und Isopropylpalmitat bestehenden Gruppe ausgewählt ist.

6. Haut-Zubereitung nach Anspruch 1 für die Behandlung trockener Haut, die eine wäßrige Mischung ist, welche 1 bis 4 Gew.-% Lecithin, das gewünschtenfalls durch eine lecithinhaltige Mischung ersetzt ist, 0,2 bis 3 Gew.-% Triäthanolamin, 2 bis 6 Gew.-% Erweichungsmittel, das aus der aus Polyoxypropylen-Fett-Äther, Isopropylpalmitat und einer Mischung derselben bestehenden Gruppe ausgewählt ist, 0,5 bis 2 Gew.-% eines Verdickungsmittels und 0,1 bis 2,0 Gew.-% Zitronensäure enthält.

7. Haut-Zubereitung nach Anspruch 6, dadurch gekennzeichnet, daß das Lecithin durch eine lecithinhaltige Phosphatid-Mischung ersetzt ist, die aus einer Sojalecithin-Mischung besteht, die 40% Lecithin, andere Phosphatide und Sojaöl enthält.

8. Haut-Zubereitung nach Anspruch 6 oder 7, dadurch gekennzeichnet, daß das Verdickungsmittel Carboxymethylcellulose ist.

9. Haut-Zubereitung nach einem der Ansprüche 6 bis 8, dadurch gekennzeichnet, daß die Zubereitung zusätzlich 15 bis 25 Gew.-% Äthanol umfaßt.

10. Haut-Zubereitung nach einem der Ansprüche 6 bis 9, die zusätzlich einen Duftstoff umfaßt.

11. Arzneimittel-Depot-System nach Anspruch 1 zur örtlichen Anwendung eines Arzneimittels auf der Haut eines Patienten, das im wesentlichen aus einer wäßrigen Mischung besteht, welche 3 bis 5 Gew.-% Lecithin, das gewünschtenfalls durch eine lecithinhaltige Mischung ersetzt ist, 0,2 bis 3 Gew.-% Triäthanolamin und eine Arzneimittel enthält, das örtlich am Patient angewandt werden soll.

12. Arzneimittel-Depot-System nach Anspruch 11, dadurch gekennzeichnet, daß das Lecithin oder die lecithinhaltige Phosphatid-Mischung in der wäßrigen Mischung in einem Anteil von 4 Gew.-% bezogen auf die Mischung vorliegt.

13. Arzneimittel-Depot-System nach Anspruch 11 oder 12, dadurch gekennzeichnet, daß eine lecithinhaltige Phosphatid-Mischung als Lecithin-Bestandteil des Arzneimittel-Depot-Systems verwendet wird, wobei die Mischung aus einer Sojalecithin-Mischung besteht, die 40% Lecithin, andere Phosphatide und Sojaöl enthält.

14. Arzneimittel-Depot-System nach einem der Ansprüche 11 bis 13, dadurch gekennzeichnet, daß das Arzneimittel in der wäßrigen Mischung in einem Anteil von bis zu 1 Gew.-% vorliegt.

15. Arzneimittel-Depot-System nach einem der Ansprüche 11 bis 14, dadurch gekennzeichnet, daß das Triäthanolamin in einem Anteil von 0,4 Gew.-% vorliegt.

16. Arzneimittel-Depot-System nach einem der Ansprüche 11 bis 15, dadurch gekennzeichnet, daß das Arzneimittel Hydrocortison ist.

17. Arzneimittel-Depot-System nach einem der Ansprüche 11 bis 16, dadurch gekennzeichnet, daß das Arzneimittel ein labiles Arzneimittel ist und die wäßrige Mischung 2,6-Di-tert-butyl-p-Kresol (DBPC) in einem Anteil von 0,02 bis 0,2% als Antioxydans zum Schutz des labilen Arzneimittels enthält.

18. Brand-Zubereitung nach Anspruch 1 zum Schutz eines Verbrennungsopfers vor Flüssigkeitsverlust durch örtliche Anwendung der Zubereitung auf der Haut des Verbrennungsopfers zur Bildung eines Filmes auf der Haut, wobei die Zubereitung im wesentlichen aus einer wäßrigen Mischung besteht, die 1 bis 5 Gew.-% Lecithin, das gewünschtenfalls durch eine lecithinhaltige Mischung ersetzt ist, und 0,2 bis 3 Gew.-% Triäthanolamin enthält.

## Patentansprüche für den Vertragsstaat: AT

1. Verfahren zur Herstellung einer Haut-Zubereitung (-Zusammensetzung), dadurch gekennzeichnet, daß man 1 bis 5 Gew.-% eines Lecithin-Bestandteiles, der aus der aus Lecithin und lecithinhaltigen Phosphatid-Mischungen bestehenden Gruppe ausgewählt ist, wobei mindestens 40% der Phosphatid-Mischung aus Lecithin besteht, mindestens eine äquimolare Menge Triäthanolamin bezogen auf das Lecithin, was 0,2 bis 3 Gew.-% Triäthanolamin entspricht, und Wasser miteinander zur Bildung einer wäßrigen Mischung vermischt, in welcher der Lecithin-Bestandteil und das Triäthanolamin in den oben angegebenen Prozentsätzen, bezogen auf die Gesamt-Zubereitung oder die wäßrige Mischung, vorliegen.

2. Verfahren nach Anspruch 1, dadurch gekennzeichnet, daß man eine Sojalecithin-Mischung, die 40% Lecithin, andere Phosphatide und Sojaöl enthält, als Lecithin-Bestandteil zum Zusammenmischen mit dem Triäthanolamin und dem Wasser zur Bildung der wäßrigen Mischung auswählt.

3. Verfahren nach Anspruch 1, bei welchem man 1 bis 4 Gew.-% Lecithin, das gewünschtenfalls durch eine lecithinhaltige Mischung ersetzt ist, 0,2 bis 3 Gew.-% Triäthanolamin mit der Maßgabe, daß mindestens eine äquimolare Menge Triäthanolamin benzogen auf die eingesetzte Menge Lecithin eingesetzt wird, 2 bis 6 Gew.-% eines Erweichungsmittels, das aus der aus Polyoxypropylen-Fett-Äther, Isopropylpalmitat und Mischungen derselben bestehenden Gruppe ausgewählt ist, 0,5 bis 2 Gew.-% eines Verdickungsmittels, 0,1 bis 2 Gew.-% Zitronensäure und den Rest der wäßrigen Zubereitung ausmachendes Wasser zusammenmischt, wobei die letztgenannten Bestandteile in den angegebenen Gewichts-Prozentsätzen bezogen auf die Gesamt-Zubereitung einschließlich des Auffüll-Wassers eingesetzt werden.

4. Verfahren nach Anspruch 3, dadurch gekennzeichnet, daß der eingesetzte Lecithin-Bestandteil eine Sojalecithin-Mischung ist, die 40% Lecithin, andere Phosphatide und Sojaöl enthält.

5. Arzneimittel-Depot-System, hergestellt nach Anspruch 1, zur örtlichen Anwendung eines Arzneimittels auf der Haut eines Patienten, das im wesentlichen aus einer wäßrigen Mischung besteht, welche 3 bis 4 Gew.-% Lecithin, das gewünschtenfalls durch eine lecithinhaltige Mischung ersetzt ist, 0,2 bis 3 Gew.-% Triäthanolamin und ein Arzneimittel enthält, das örtlich am Patient angewandt werden soll.

6. Arzneimittel-Depot-System nach Anspruch 5, dadurch gekennzeichnet, daß das Lecithin oder die lecithinhaltige Phosphatid-Mischung in der wäßrigen Mischung in einem Anteil von 4 Gew.-% bezogen auf die Mischung vorliegt.

7. Arzneimittel-Depot-System nach Anspruch 5 oder 6, dadurch gekennzeichnet, daß eine lecithinhaltige Phosphatid-Mischung als Lecithin-Bestandteil des Arzneimittel-Depot-Systems verwendet wird, wobei die Mischung aus einer Sojalecithin-Mischung besteht, die 40% Lecithin, andere Phosphatide und Sojaöl enthält.

8. Arzneimittel-Depot-System nach einem der Ansprüche 5 bis 7, dadurch gekennzeichnet, daß das Arzneimittel in der wäßrigen Mischung in einem Anteil von bis zu 1 Gew.-% vorliegt.

9. Arzneimittel-Depot-System nach einem der Ansprüche 5 bis 8, dadurch gekennzeichnet, daß das Triäthanolamin in einem Anteil von 0,4 Gew.-% vorliegt.

10. Arzneimittel-Depot-System nach einem der Ansprüche 5 bis 9, dadurch gekennzeichnet, daß das Arzneimittel Hydrocortison ist.

11. Arzneimittel-Depot-System nach einem der Ansprüche 5 bis 10, dadurch gekennzeichnet, daß das Arzneimittel ein labiles Arzneimittel ist und die wäßrige Mischung 2,6-Di-tert-butyl-p-Kresol (DBPC) in einem Anteil von 0,02 bis 0,2% als Antioxydans zum Schutz des labilen Arzneimittels enthält.

12. Brand-Zubereitung, hergestellt nach Anspruch 1, zum Schutz eines Verbrennungsopfers vor Flüssigkeitsverlust durch örtliche Anwendung der Zubereitung auf der Haut des Verbrennungsopfers zur Bildung eines Filmes auf der Haut, wobei die Zubereitung im wesentlichen aus einer wäßrigen Mischung besteht, die 1 bis 5 Gew.-% Lecithin, das gewünschtenfalls durch eine lecithinhaltige Mischung ersetzt ist, und 0,2 bis 3 Gew.-% Triäthanolamin enthält.